Europäisches Patentamt

European Patent Office

Office européen des brevets

(11) Numéro de publication : **0 583 179 A1**

## (12) DEMANDE DE BREVET EUROPEEN

(21) Numéro de dépôt : 93401722.9

(51) Int. Cl.⁵ : **C09K 5/04**

(22) Date de dépôt : 02.07.93

(30) Priorité : 12.08.92 FR 9209962

(43) Date de publication de la demande :
16.02.94 Bulletin 94/07

(84) Etats contractants désignés :
AT BE CH DE DK ES FR GB GR IE IT LI LU NL
PT SE

(72) Inventeur : **Macaudiere, Sylvie**
**64, Rue de Nanterre**
**F-92600 Asnieres (FR)**
Inventeur : **Tanguy, Jean-Claude**
**7, Rue du Lieutenant Keiser**
**F-95110 Sannois (FR)**

(71) Demandeur : **ELF ATOCHEM S.A.**
**4 & 8, Cours Michelet La Défense 10**
**F-92800 Puteaux (FR)**

(74) Mandataire : **Leboulenger, Jean et al**
**Elf Atochem S.A. Département Propriété**
**Industrielle, Cedex 42 - La Défense 10**
**F-92091 Paris la Défense (FR)**

(54) **Mélanges pseudo-azéotropiques de difluorométhane, de pentafluoroéthane et de 1,1,1-trifluoroéthane, et leur application comme fluides frigorigènes en réfrigération basse température.**

(57) Pour remplacer le mélange azéotropique de chlorodifluorométhane et de chloropentafluoroéthane (CFC 502) en réfrigération basse température, l'invention propose d'utiliser un mélange pseudo-azéotropique contenant en masse 1 à 15 % de difluorométhane, 37 à 50 % de pentafluoroéthane et 35 à 62 % de 1,1,1-trifluoroéthane.
Les propriétés thermodynamiques de ce mélange sont très proches de celles du mélange CFC 502.

EP 0 583 179 A1

La présente invention concerne le domaine de la réfrigération et a plus particulièrement pour objet des mélanges pseudo-azéotropiques de fluides frigorigènes à bas point d'ébullition, n'ayant pas ou peu d'action sur l'environnement, pour remplacer les chlorofluorocarbures (CFC) dans les systèmes de réfrigération basse température.

Dans ces systèmes exploités en réfrigération commerciale ou industrielle, on opère selon un cycle thermodynamique défini généralement par une température d'évaporation comprise entre -35 et -45°C (le plus souvent -40°C), une température de condensation comprise entre +30 et +55°C, un sous-refroidissement liquide de l'ordre de -5°C et une surchauffe des vapeurs d'au moins 10°C.

Le fluide frigorigène couramment utilisé en réfrigération basse température est le mélange azéotropique (appelé CFC 502) de chlorodifluorométhane et de chloropentafluoroéthane. Or, il est maintenant établi qu'à cause de leur coefficient important d'action sur l'ozone, les CFC et en particulier le CFC 502, devront à plus ou moins longue échéance être remplacés par des fluides frigorigènes ne contenant plus de chlore et, de ce fait, moins agressifs vis-à-vis de l'environnement.

Pour remplacer le CFC 502 dans les installations de réfrigération basse température existantes, le substitut doit présenter des propriétés thermodynamiques, en particulier un coefficient de performance (COP) et une capacité frigorifique, aussi proches que possible de celles du CFC 502. D'autre part, pour la bonne stabilité du produit et la durabilité du matériel, il est souhaitable que la température de refoulement du substitut n'excède pas celle du CFC 502 de plus de 5°C environ. Enfin, le substitut doit être ininflammable et le rester en cas de fuite en phase vapeur.

La demande de brevet EP 0 451 692 revendique un réfrigérant composé d'un mélange de trois hydrofluorocarbures de formule $C_lH_mF_n$ où les indices l, m et n ont les significations suivantes:

| l | m | n |
|---|---|---|
| 1 | 1 ou 2 | 2 ou 3 |
| 2 | 1 à 4 | 2 à 5 |
| 3 | 1 à 3 | 5 à 7 |

Cependant, cette demande de brevet vise essentiellement le remplacement du chlorodifluorométhane (HCFC 22) en réfrigération moyenne température (0°C) et ne contient aucune indication utile pour la substitution du CFC 502 en réfrigération basse température.

Le difluorométhane (HFC 32), le pentafluoroéthane (HFC 125) et le 1,1,1-trifluoroéthane (HFC 143a) ont comparativement aux composés totalement halogénés, une très faible action sur l'environnement.

Le HFC 143a, bien qu'aussi efficace que le CFC 502, présente cependant l'inconvénient majeur d'être inflammable.

Le HFC 32, lui aussi inflammable, possède de plus une température de refoulement en sortie de compresseur très supérieure à celle du CFC 502. Il est connu par l'homme de l'art qu'une température de refoulement trop élevée est néfaste à la bonne stabilité du produit et à la durabilité du matériel.

Quant au HFC 125, composé ininflammable, il peut être considéré comme substitut possible au CFC 502. Cependant, il présente le gros désavantage d'être nettement moins efficace que le CFC 502.

Il a maintenant été trouvé que les mélanges contenant en masse environ 1 à 15 % de HFC 32, 37 à 50 % de HFC 125 et 35 à 62 % de HFC 143a, présentent, comparativement aux composés individuels, des propriétés thermodynamiques très proches de celles du CFC 502. Les mélanges selon l'invention possèdent de plus un coefficient d'action sur l'ozone nul et un effet de serre plus faible que celui du CFC 502. Ils présentent d'autre part un comportement pseudo-azéotropique, ce qui minimise les problèmes de distillation dans le circuit et de maintenance. Enfin, au contraire des HFC 32 et 143a, les mélanges selon l'invention sont ininflammables à température ambiante et le restent même en cas de fuites de la phase vapeur.

Les mélanges selon l'invention peuvent donc être utilisés dans les systèmes de réfrigération basse température, notamment pour le remplacement du CFC 502 en réfrigération industrielle ou commerciale.

Parmi les mélanges selon l'invention, un mélange tout particulièrement préféré contient environ 10 % de HFC 32, 45 % de HFC 125 et 45 % de HFC 143a.

Les exemples suivants illustrent l'invention sans la limiter.

## EXEMPLE 1

Cet exemple montre que les mélanges HFC 32/HFC 125/HFC 143a selon l'invention ne subissent qu'une très faible distillation et maintiennent constante leur tension de vapeur, au cours d'une fuite de la phase vapeur.

La température a été maintenue constante à 24°C. Les pressions ont été mesurées au moyen d'un manomètre Heise avec une précision supérieure à ± 1 %. Le récipient est initialement chargé avec environ 200 g d'un mélange contenant 10 % de HFC 32, 45 % de HFC 125 et 45 % de HFC 143a.

La fuite en phase vapeur est poursuivie jusqu'à ce que 80 % de la charge initiale soit dissipée. Durant l'expérience, des échantillons de la phase gaz sont collectés et analysés par les moyens standard de chromatographie phase gaz. La tension de vapeur est également mesurée au même moment. Les résultats obtenus sont rassemblés dans le tableau suivant.

**TABLEAU I**

| Taux de vidange (%) | Pression absolue (bar) | Composition (% masse) | | |
|---|---|---|---|---|
| | | HFC 32 | HFC 125 | HFC 143a |
| 0 | 14,57 | 13,9 | 43,3 | 42,7 |
| 2,8 | 14,57 | 14,5 | 43,2 | 42,2 |
| 9,6 | 14,57 | 15,2 | 42,9 | 41,9 |
| 20,0 | 14,57 | 16,1 | 42,7 | 41,2 |
| 33,8 | 14,34 | 12,3 | 43,4 | 44,3 |
| 41,2 | 14,35 | 12,5 | 43,3 | 44,2 |
| 55,1 | 14,26 | 11 | 43,7 | 45,3 |
| 67,2 | 14,2 | 10,1 | 43,8 | 46 |
| 78,7 | 14,07 | 8,5 | 44 | 47,5 |

Ces données montrent qu'une perte de près de 80 % de la charge initiale en poids ne modifie que faiblement la composition du mélange et maintient quasiment constante la tension de vapeur du mélange à ± 4 %. Un tel mélange peut être considéré comme pseudo-azéotropique.

D'autre part, les limites d'inflammabilité dans l'air de différents mélanges binaires et ternaires de HFC 32, HFC 125 et HFC 143a ont été déterminées suivant la méthode ASTM E-681. Qu'il s'agisse des mélanges binaires HFC 32/HFC 125 et HFC 143a/HFC 125 ou des mélanges ternaires HFC 32/HFC 125/HFC 143a et quelle que soit la proportion respective de HFC 32 et HFC 143a, la limite d'inflammabilité dans l'air à température ambiante des différents mélanges correspond à une teneur maximale en masse de 63 % en HFC 32 et HFC 143a.

Les mélanges ternaires selon l'invention sont donc ininflammables et le restent même lors d'une fuite de vapeur puisque, comme le montre le tableau I, la teneur totale en HFC 32 et HFC 143a n'est que de 56 % après une fuite d'environ 80 %.

## EXEMPLE 2

Cet exemple montre que la tension de vapeur des mélanges pseudo-azéotropiques de HFC 32/HFC 125/HFC 143a est proche de celle du CFC 502 et ce sur une large gamme de température.

Le tableau II rassemble les données pour un mélange contenant en masse 10 % de HFC 32, 45 % de HFC 125 et 45 % de HFC 143a.

## TABLEAU II

| Température (°C) | Pression absolue (bar) | |
|---|---|---|
| | Mélange selon l'invention | CFC 502 |
| -50 | 0,99 | 0,814 |
| -30 | 2,35 | 1,979 |
| -10 | 4,89 | 4,143 |
| 10 | 9,18 | 7,73 |
| 20 | 12,17 | 10,2 |
| 30 | 15,83 | 13,19 |
| 50 | 25,45 | 21,01 |

### EXEMPLE 3

Cet exemple illustre l'utilisation des mélanges selon l'invention comme fluides frigorigènes.

Les performances thermodynamiques de différents mélanges selon l'invention ont été comparées aux performances des constituants seuls et à celles du CFC 502, pour un cycle thermodynamique standard défini comme suit:

Température de condensation        : + 30°C
Température d'évaporation            : - 40°C
Sous-refroidissement liquide         : - 5°C
Surchauffe des vapeurs               : + 25°C

Le tableau III résume les performances thermodynamiques observées dans ces conditions pour le HFC 32, HFC 125, HFC 143a et leurs mélanges.

EP 0 583 179 A1

## TABLEAU III

| Composition HFC 32/HFC 125/HFC 143a (% masse) | COP (*) | Capacité frigorifique volumétrique (*) | Différence de température (K) de refoulement (*) |
|---|---|---|---|
| 0/100/0 | 0,93 | 1,03 | - 15 |
| 100/0/0 | 0,96 | 1,47 | 72 |
| 0/0/100 | 0,98 | 1,02 | 0 |
| 46/54/0 | 0,97 | 1,42 | 22 |
| 60/0/40 | 0,97 | 1,48 | 39 |
| 0/45/55 | 0,97 | 1,00 | - 6 |
| 20/45/35 | 0,96 | 1,18 | 7 |
| 20/40/40 | 0,96 | 1,18 | 8 |
| 15/40/45 | 0,96 | 1,13 | 5 |
| 10/45/45 | 0,96 | 1,09 | 1 |
| 7,5/42,5/50 | 0,96 | 1,07 | - 1 |
| 5/47,5/47,5 | 0,96 | 1,04 | - 3 |

(*) Valeurs rapportées au CFC 502

Le tableau IV résume les mêmes données, mais pour une température de condensation de 55°C, les autres conditions étant inchangées.

## TABLEAU IV

| Composition HFC 32/HFC 125/HFC 143a (% masse) | COP (*) | Capacité frigorifique volumétrique (*) | Différence de température (K) de refoulement(*) |
|---|---|---|---|
| 0/100/0 | 0,76 | 0,83 | - 18 |
| 100/0/0 | 1,03 | 1,64 | 91 |
| 0/0/100 | 0,92 | 0,97 | 0 |
| 10/45/45 | 0,89 | 1,02 | 1 |
| 15/40/45 | 0,91 | 1,08 | 5 |

(*) Valeurs rapportées au CFC 502

**Revendications**

1. Mélange pseudo-azéotropique contenant en masse 1 à 15 % de difluorométhane, 37 à 50 % de pentafluoroéthane et 35 à 62 % de 1,1,1-trifluoroéthane.

5

2. Mélange selon la revendication 1 contenant en masse environ 10 % de difluorométhane, 45 % de pentafluoroéthane et 45 % de 1,1,1-trifluoroéthane.

3. Utilisation d'un mélange selon la revendication 1 ou 2 comme fluide frigorigène en réfrigération basse température.

Office européen
des brevets

**RAPPORT DE RECHERCHE EUROPEENNE**

Numero de la demande

EP 93 40 1722

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| D,A | EP-A-0 451 692 (DAIKIN INDUSTRIES)<br>* revendication 1; figure 8; exemple 8 *<br>--- | 1 | C09K5/04 |
| A | US-A-4 943 388 (I.R. SHANKLAND, R.G. RICHARD, E.A.E. LUND)<br>* colonne 4, ligne 3 - ligne 50 *<br>* colonne 8, ligne 56 - colonne 9, ligne 19 *<br>* revendications 1,2; exemple 3; tableaux 3,4 *<br>--- | 1,3 | |
| A | WO-A-92 11338 (DU PONT DE NEMOURS AND COMPANY)<br>* page 3, ligne 24 - page 4, ligne 18 *<br>* page 7, ligne 24 - ligne 30; tableau 2 *<br>--- | 1,3 | |
| A | WO-A-92 11339 (DU PONT DE NEMOURS AND COMPANY)<br>* page 3, ligne 6 - ligne 20 *<br>* page 5, ligne 11 - ligne 15; tableau 2 *<br>----- | 1,3 | DOMAINES TECHNIQUES RECHERCHES (Int.Cl.5)<br><br>C09K |

Le présent rapport a été établi pour toutes les revendications

| Lieu de la recherche | Date d'achèvement de la recherche | Examinateur |
|---|---|---|
| LA HAYE | 5 Novembre 1993 | PUETZ, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...........................................................
& : membre de la même famille, document correspondant

EPO FORM 1503 03.82 (P04C02)